# FASCICULE DE BREVET EUROPEEN

(11) **EP 2 393 773 B1**
(45) Date de publication et mention de la délivrance du brevet: **17.09.2014**
(21) Numéro de dépôt: 10708286.9
(22) Date de dépôt: 05.02.2010
(51) Int. Cl.: C07C 227/06, C07C 229/08, C07C 253/22, C07C 253/30, C07C 51/353, C12P 7/44

(54) **PROCÉDÉ DE SYNTHÈSE D'UN OMEGA-AMINOACIDE OU ESTER À PARTIR D'UN ACIDE OU ESTER GRAS MONO-INSATURÉ**
VERFAHREN ZUR HERSTELLUNG EINER OMEGA-AMINOSÄURE ODER EINES ESTERS AUS EINER EINFACH UNGESÄTTIGTEN FETTSÄURE ODER EINEM FETTSÄUREESTER
METHOD FOR SYNTHESIZING AN OMEGA-AMINO ACID OR ESTER FROM A MONOUNSATURATED FATTY ACID OR ESTER

(30) Priorité: 05.02.2009 FR 0950704
(43) Date de publication de la demande: 14.12.2011
(73) Titulaire: Arkema France, 92700 Colombes (FR)
(72) Inventeur: DUBOIS, Jean-Luc, F-69390 Millery (FR)
(86) Numéro de dépôt international: PCT/FR2010/050186
(87) Numéro de publication internationale: WO 2010/089512

(56) Documents cités:
- WO-A-2008/104722
- GB-A- 741 739
- US-A- 3 974 196
- R. B. PERKINS JR. ET AL.: "Nylon-9 from Unsaturated Fatty Derivatives: Preparation and Characterization" JOURNAL OF THE AMERICAN OIL CHEMISTS' SOCIETY., vol. 52, novembre 1975 (1975-11), pages 473-477, XP002545488 SPRINGER, BERLIN. cité dans la demande
- HELEN L.NGO ET AL.: "Metathesis of Unsaturated Fatty Acids: Synthesis of Long-Chain Unsaturated-alpha,omega-Dicarboxylic Acids" JOURNAL OF THE AMERICAN OIL CHEMISTS' SOCIETY., vol. 83, no. 7, 2006, pages 629-634, XP002545489 SPRINGER, BERLIN.

## Description

L'invention vise un procédé de synthèse d'acides ω-amino-alcanoïques ou de leurs esters à partir d'acides gras naturels mono-insaturés transitant par un composé intermédiaire de type dinitrile mono-insaturé.

L'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amide -CO-NH-, C'est ainsi que sont connus les Nylon-6, Nylon 6-6, Nylon 6-10, Nylon 7, Nylon 8, Nylon 9, Nylon 11, Nylon 13, etc.

Ces monomères sont par exemple fabriqués par voie de synthèse chimique en utilisant notamment comme matière première des oléfines en C2 à C4, des cycloalcanes ou du benzène, mais aussi de l'huile de ricin (Nylon 11), de l'huile érucique ou lesquérolique (Nylon 13).

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide amino-11-undécanoïque, qui est à la base de la synthèse du Rilsan 11 ^{®}. Ce procédé est décrit dans l'ouvrage « Les Procédés de Pétrochimie » de A. Chauvel et al. paru aux Editions TECHNIP (1986). L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part l'undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.

Les principaux travaux de recherche ont porté sur la synthèse de l'acide amino-9-nonanoïque qui est le précurseur du Nylon 9 à partir de l'acide oléique d'origine naturelle.

En ce qui concerne ce monomère particulier on peut citer *l'ouvrage* « n-Nylons, Their Synthesis, Structure and Properties » - 1997 Ed. J. Wiley et Sons dont le chapitre 2.9 (pages 381 à 389) est consacré au 9-Nylon. Cet article donne la synthèse des réalisations et des travaux conduits sur le sujet. On y mentionne, page 381, le procédé développé en ex Union Soviétique ayant conduit à la commercialisation du Pelargon®. On y mentionne, page 384, également un procédé développé au Japon utilisant l'acide oléique venant l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.

Pour être complet sur l'état de l'art en la matière, il faut citer les nombreux articles publiés par E. H. Pryde et al. entre 1962 et 1975 dans - Journal of the American Oil Chemists 'Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et R.B. Perkins et al. « Nylon-9 from Unsaturated Fatty Derivatives : Préparation and Characterization » JAOCS, Vol. 52 pages 473-477. Il est à noter que le premier de ces articles fait aussi référence page 498 à des travaux antérieurs réalisés par des Japonais : H. Otsuki et H. Funahashi.

Pour résumer cette partie de l'état de l'art visant ce type de synthèse du « Nylon 9 » à partir d'huiles végétales on peut décrire le mécanisme réactionnel simplifié suivant appliqué à l'ester oléique, extrait des huiles par méthanolyse :
Ozonolyse réductrice

   H₃C-(CH₂)₇-CH=CH-(CH₂)₇-COOCH₃ + (O₃, H₂) → HOC-(CH₂)₇-COOCH3 + H₃C-(CH₂)₇-COH
Amination réductrice

   HOC-(CH₂)₇-COOCH₃ + (NH₃, H₂) → H₂N-(CH₂)₈-COOCH₃ + H₂O
Hydrolyse

   HOC-(CH₂)₈-COOCH₃ +H₂O → H₂N-(CH₂)₈-COOH + CH₃OH

Cette voie très séduisante sur le plan réactionnel présente cependant un inconvénient économique important constitué par la production lors de la première étape d'un aldéhyde à longue chaîne (9 atomes de carbone au total) pratiquement non valorisable notamment dans l'industrie des polymères de type polyamides.

Le brevet UK n° 741,739 décrit pour sa part la synthèse de ce même acide à partir de l'acide oléique mais en utilisant la voie oléonitrile. Le schéma réactionnel simplifié de ce procédé est le suivant. Une voie analogue est citée dans l'article de R.B. Perkins et al cité ci-dessus p. 475.

H₃C-(CH₂)₇-CH=CH-(CH₂)₇-COOH + NH₃ → H₃C-(CH₂)₇-CH=CH-(CH₂)₇-CN + 2 H₂O

H₃C-(CH₂)₇-CH=CH-(CH₂)₇-CN + (O₃ + H₂O Ozonolyse oxydante)→ H₃C-(CH₂)₇-COOH + NC-(CH₂)₇-COOH

NC-(CH₂)₇-COOH + 2 H₂ → H₂N-(CH₂)₈-COOH

Cette synthèse conduit à l'acide pélargonique H₃C-(CH₂)₇-COOH comme sous-produit.

La présente invention vise à proposer un nouveau procédé pour synthétiser toute une gamme d'acides ω-amino-alcanoïques ou de leurs esters à partir d'acides gras naturels mono-insaturés.

Le problème est donc de trouver un procédé de synthèse de divers ω-amino-acides de formule H₂N-(CH₂)ₙ-COOH dans laquelle n est compris entre 3 et 14 (et de leur polymères) à partir de matières premières renouvelables, de très large accès, et donc peu onéreuses, simple à mettre en oeuvre tout en évitant d'une part les contraintes environnementales évoquées précédemment et d'autre part les handicaps économiques dus aux sous-produits des réactions.

La solution proposée consiste à travailler à partir de matières premières constituées par des acides gras insaturés à longue chaîne naturels comportant le cas échéant une fonction hydroxyle, à les transformer dans un premier stade en dinitriles mono-insaturés puis ensuite dans un deuxième stade à « réinsérer » dans le produit final une fonction acide carboxylique par action sur la double liaison du dinitrile mono-insaturé provoquant une coupure de la molécule de dinitrile, soit au moyen d'une coupure oxydante, soit par réaction de métathèse croisée avec un composé de type acrylate.

On entend par acide gras naturel un acide issu des milieux végétal ou animal, y compris les algues, plus généralement du règne végétal, et donc renouvelable. Cet acide constitué d'au moins 10 et de préférence d'au moins 14 atomes de carbone par molécule comportera au moins une insaturation oléfinique dont la localisation en position x par rapport au groupement acide (delta x) déterminera la formule de l'ω-amino-acide final. En outre cet acide gras naturel pourra, le cas échéant, comporter une fonction hydroxyle.

On peut citer à titre d'exemples de tels acides, les acides en C10, les acides obtusilique (cis-4-décénoique) et caproléique (cis-9-décénoique), les acides en C12, les acides lauroléique (cis-5-dodécénoique) et lindérique (cis-4-dodécénoique), les acides en C14, les acides myristoléique (cis-9-tétradécénoique), physétérique (cis-5-tetradécénoique) et tsuzuique (cis-4-tetradécénoique), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoique) et ricinoléique (12-hydroxy--cis-9-octadécénoïque), les acides en C20, les acides gadoléique (cis-9-eicosénoïque), gondoïque (cis-11-eicosénoïque), cis-5-eicosénoïque et lesquérolique (14-hydroxy-cis-11-eicosénoïque), les acides en C22, les acides cétoléique (cis-11-docosénoïque) et érucique (cis-13-docosénoïque).

Le procédé peut également s'appliquer aux acides poly-insaturés tels que les acides linoléiques (cis,cis-9,12-octadécadiénoïque et cis,trans-9,11-octadécadiénoïque), α-linolénique (cis,cis,cis-9,12,15-octadécatriénoïque), α-éléostéarique (cis,trans,trans-9,11,13-octadécatriénoïque) mais avec l'inconvénient de multiplier les sous-produits. Ces divers acides sont issus des huiles végétales extraites de diverses plantes oléagineuses telles que le tournesol, le colza, le ricin, le lesquerella, l'olive, le soja, le palmier, l'avocat, l'argousier, la coriandre, le céleri, l'aneth, la carotte, le fenouil, le Limnanthes Alba (meadowfoam), le carthame, la caméline

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise un procédé de synthèse d'un ω-aminoacide(ester) de formule ROOC-(CH₂)q-CH₂-NH₂ dans laquelle R est H ou un radical alkyle comportant de 1 à 4 atomes de carbone et q un indice entier égal soit à p ou à p+2, soit à n ou n+2, compris entre 2 et 15, à partir d'un acide(ester) gras mono-insaturé de formule R₁-CH=CH-(CH₂)ₚ-COOR₂ dans laquelle R₁ est soit H, soit un radical alkyle comprenant de 4 à 14 atomes de carbone et le cas échéant une fonction hydroxyle, R₂ est H ou un radical alkyle comportant de 1 à 4 atomes de carbone et p un indice entier compris entre 2 et 11, comportant une étape réactionnelle d'ammoniation conduisant à la transformation de la fonction carbonyle en fonction nitrile caractérisé en ce que
dans un premier stade on transforme l'acide/ester gras insaturé en un dinitrile insaturé de formule NC-(CH₂)ₚ-CH=CH-(CH₂)ₙ-CN dans laquelle n est un entier compris entre 3 et 13 dépendant de la nature du radical R₁, en deux étapes successives, la première étape étant soit une homométathèse de l'acide gras conduisant au diacide insaturé symétrique de formule R₂OOC-(CH₂)ₚ-CH=CH-(CH₂)ₚ-COOR₂, soit une fermentation de cet acide/ester, conduisant à un diacide insaturé de formule HOOC-(CH₂)ₚ-CH=CH-(CH₂)ₙ-COOH (la fermentation donnera des diacides - l'alcool est consommé) et la seconde une ammoniation des acides puis,
dans un deuxième stade on transforme ce dinitrile insaturé en un nitrile acide/ester de formule R₃OOC- [CH=CH]ₓ (CH₂)_{p,n}-CN dans laquelle R₃ est H ou un radical alkyle comportant de 1 à 4 atomes de carbone, x est 0 ou 1, « p,n » signifiant que l'indice est soit p, soit n selon la voie choisie lors du premier stade, transformation réalisée soit par coupure oxydante du dinitrile insaturé, soit par une réaction de métathèse croisée du dinitrile insaturé avec un acrylate de formule CH₂=CH-COOR₃ et,
dans un troisième stade à hydrogéner le nitrile acide en ω-aminoacide(ester) de formule R₃OOC-(CH₂)_{q}-CH₂NH₂.

Dans une variante du procédé mettant en oeuvre la voie homométathèse lors du premier stade, il est possible d'intervertir les étapes d'ammoniation conduisant au nitrile et de métathèse s'appliquant là au nitrile gras pour passer au dinitrile.

Le processus réactionnel du cas général est alors le suivant.
1) premier stade :
   soit homométathèse

      2 R₁-CH=CH-(CH₂)ₚ-COOH ⇔ R₁-CH=CH- R₁ + HOOC-(CH₂)ₚ-CH=CH-(CH₂)ₚ-COOH
   soit fermentation

      R₁-CH=CH-(CH₂)ₚ-COOH (oxydation) → HOOC-(CH₂)ₙ-CH=CH-(CH₂)ₚ-COOH

      puis

      HOOC-(CH₂)ₚ-CH=CH-(CH₂)ₚ-COOH + 2 NH₃ → NC-(CH₂)ₚ-CH=CH-(CH₂)ₚ-CN + 4 H₂O

      ou

      HOOC-(CH₂)ₙ-CH=CH-(CH₂)ₚ-COOH + 2 NH₃ → NC-(CH₂)ₙ-CH=CH-(CH₂)ₚ-CN + 4 H₂O
2) deuxième stade :
   première variante (coupure oxydante)

      NC-(CH₂)ₚ-CH=CH-(CH₂)ₚ-CN + (coupure oxydante) → 2 HOOC-(CH₂)ₚ-CN

      ou

      NC-(CH₂)ₙ-CH=CH-(CH₂)ₚ-CN + (coupure oxydante) → HOOC-(CH₂)ₚ-CN + COOH-(CH₂)ₙ-CN

      seconde variante (métathèse croisée)

      NC-(CH₂)ₚ-CH=CH-(CH₂)ₚ-CN + 2 CH₂=CH-COOR₃ ⇔ 2 R₃OOC-CH=CH-(CH₂)ₚ-CN + CH₂=CH₂

   NC-(CH₂)ₙ-CH=CH-(CH₂)ₚ-CN + 2 CH₂=CH-COOR₃ ⇔ R₃OOC-CH=CH-(CH₂)ₚ-CN + NC-(CH₂)ₙ-CH=CH-COOR₃ + CH₂=CH₂
3) troisième stade :
   première variante après coupure oxydante

      2 HOOC-(CH₂)ₚ-CN +4 H₂ → 2 HOOC-(CH₂)ₚ-CH₂NH₂

      HOOC-(CH₂)ₚ-CN + HOOC-(CH₂)ₙ-CN + 4H₂ → HOOC-(CH₂)ₚ-CH₂NH₂ + HOOC-(CH₂)ₙ-CH₂NH₂
   seconde variante après métathèse

      R₃OOC-CH=CH-(CH₂)ₚ-CN + 3 H₂ → R₃OOC-(CH₂)ₚ₊₂-CH₂NH₂

      R₃OOC-CH=CH-(CH₂)ₚ-CN + NC-(CH₂)ₙ-CH=CH-COOR₃ + 6 H₂ →

      R₃OOC-(CH₂)ₚ₊₂-CH₂NH₂ + R₃OOC-(CH₂)ₙ₊₂-CH₂NH₂

Dans le schéma réactionnel précédent, comme pour les suivants, lorsqu'il est indiqué que la réaction met en jeu la forme acide du composé, elle peut tout aussi bien s'appliquer à sa forme ester.

Ces divers mécanismes sont illustrés par le diagramme schématique (schéma 1) figurant ci-après dans la description.

Appliqués à l'acide oléique pour lequel n est égal à p on obtient R₃OOC-(CH₂)₉-CH₂NH₂ par la voie métathèse croisée et R₃OOC-(CH₂)₇-CH₂NH₂ par la voie coupure oxydante.

Appliqués à un acide dont la double liaison n'est pas localisée au centre de la molécule, tel que l'acide palmitoléique, les processus deviennent
i)

   CH₃-(CH₂)₅-CH=CH-(CH₂)₇-COOH + NH₃ → CH₃-(CH₂)₅-CH=CH-(CH₂)₇-CN + 2 H₂O

   2 CH₃-(CH₂)₅-CH=CH-(CH₂)₇-CN ⇔ NC-(CH₂)₇-CH=CH-(CH₂)₇-CN + CH₃-(CH₂)₅-CH=CH-(CH₂)₅-CH₃

   NC-(CH₂)₇-CH=CH-(CH₂)₇-CN + (coupure oxydante) → 2 NC-(CH₂)₇-COOH + HCHO/HCOOH

   NC-(CH₂)₇-COOH + 2 H₂→ HOOC-(CH₂)₇-CH₂NH₂
ii)

   CH₃-(CH₂)₅-CH=CH-(CH₂)₇-COOH + NH₃ → CH₃-(CH₂)₅-CH=CH-(CH₂)₇-CN + 2 H₂O

   2 CH₃-(CH₂)₅-CH=CH-(CH₂)₇-CN ⇔ NC-(CH₂)₇-CH=CH-(CH₂)₇-CN + CH₃-(CH₂)₅-CH=CH-(CH₂)₅-CH₃

   NC-(CH₂)₇-CH=CH-(CH₂)₇-CN + 2 CH₂=CH-COOR₃ ⇔ 2 NC-(CH₂)₇-CH=CH-COOR₃ + CH₂=CH₂

   NC-(CH₂)₇-CH=CH-COOR₃ + 3 H₂ → R₃OOC-(CH₂)₉-CH₂NH₂
iii)

   CH₃-(CH₂)₅-CH=CH-(CH₂)₇-COOH (oxydation par fermentation) → HOOC-(CH₂)₅-CH=CH-(CH₂)₇-COOH

   HOOC-(CH₂)₅-CH=CH-(CH₂)₇-COOH + 2 NH₃ → NC-(CH₂)₅-CH=CH-(CH₂)₇-CN + 4 H₂O

   NC-(CH₂)₅-CH=CH-(CH₂)₇-CN +(coupure oxydante) → HOOC-(CH₂)₅-CN + HOOC-(CH₂)₇-CN

   HOOC-(CH₂)₅-CN + 2 H₂ → HOOC-(CH₂)₅-CH₂NH₂
iv)

   CH₃-(CH₂)₅-CH=CH-(CH₂)7-COOH (oxydation par fermentation) → HOOC-(CH₂)₅-CH=CH-(CH₂)₇-COOH

   HOOC-(CH₂)₅-CH=CH-(CH₂)₇-COOH + 2 NH₃ → NC-(CH₂)₅-CH=CH-(CH₂)₇-CN + 4 H₂O

   NC-(CH₂)₅-CH=CH-(CH₂)₇-CN + 2 CH₂=CH-COOR₃ ⇔ NC-(CH₂)₇-CH=CH-COOR₃ + NC-(CH₂)₅-CH=CH-COOR₃ + CH₂=CH₂

   NC-(CH₂)₇-CH=CH-COOR₃ + 3 H₂ → R₃OOC-(CH₂)₉-CH₂NH₂

   NC-(CH₂)₅-CH=CH-COOR₃ + 3 H₂ → R₃OOC-(CH₂)₇-CH₂NH₂

Les seuls « sous-produits » formés sont une α-oléfine à longue chaîne, comportant le cas échéant une fonction hydroxyle, et du formaldéhyde ou de l'acide formique.

Dans une première variante de réalisation du procédé, lors du premier stade on réalise tout d'abord l'homométathèse de l'acide/ester gras de formule R₁-CH=CH-(CH₂)ₚ-puis l'ammoniation du diacides/diesters gras obtenu pour obtenir un dinitrile gras, puis dans un deuxième stade on transforme ce dinitrile en nitrile-acide de formule HOOC-(CH₂)ₚ-CN par coupure oxydante et, enfin, dans un troisième stade on réduit par hydrogénation la fonction nitrile en fonction amine pour obtenir le composé de formule ROOC-(CH₂)ₚ-CH₂NH₂.

Dans une deuxième variante de réalisation du procédé, lors du premier stade on réalise tout d'abord l'homométathèse de l'acide(ester) gras de formule R₁-CH=CH-(CH₂)ₚ-COOR₂ pour obtenir le diacide/ester de formule R₂OOC--(CH₂)ₚ-CH=CH-(CH₂)ₚ-COOR₂ puis l'ammoniation du diacides/diesters gras obtenu pour obtenir un dinitrile gras, puis dans un deuxième stade on transforme ce dinitrile en nitrile-acide/ester de formule R₃OOC-[CH=CH]-(CH₂)ₚ-CN par métathèse croisée avec l'acrylate d'alkyle CH₂=CH-COO R₃ et enfin dans un troisième stade on réduit simultanément par hydrogénation la double liaison et la fonction nitrile en amine pour obtenir lé composé de formule R₃OOC-(CH₂)ₚ₊₂-CH₂NH₂.

Dans une troisième variante de réalisation du procédé, lors du premier stade on réalise tout d'abord l'ammoniation de l'acide(ester) gras de formule R₁-CH=CH-(CH₂)ₚ-COOR₂ conduisant au nitrile correspondant puis la transformation par homométathèse du nitrile en dintrile gras insaturé de formule NC-(CH₂)ₚ-CH=CH-(CH₂)ₚ-CN, puis on transforme ce dinitrile en nitrile-acide de formule HOOC-(CH₂)ₚ-CN par coupure oxydante et, enfin, dans un troisième stade on réduit par hydrogénation la fonction nitrile en fonction amine pour obtenir le composé de formule HOOC-(CH₂)ₚ-CH₂NH₂.

Dans une quatrième variante de réalisation du procédé, lors du premier stade on réalise tout d'abord l'ammoniation de l'acide(ester) gras de formule R₁-CH=CH-(CH₂)ₚ-COOR₂ conduisant au nitrile correspondant puis la transformation par homométathèse du nitrile en dintrile gras insaturé de formule NC-(CH₂)ₚ-CH=CH-(CH₂)ₚ-CN, puis dans un deuxième stade on transforme ce dinitrile en nitrile-ester de formule R₃OOC-[CH=CH] -(CH₂)ₚ-CN par métathèse croisée avec l'acrylate d'alkyle CH₂=CH-COOR₃ et enfin dans un troisième stade on réduit simultanément par hydrogénation la double liaison et la fonction nitrile en amine pour obtenir le composé de formule R₃OOC-(CH₂)ₚ₊₂-CH₂NH₂.

Dans une cinquième variante de réalisation du procédé, lors du premier stade on réalise tout d'abord l'oxydation par fermentation de l'acide/ester gras de formule R₁-CH=CH-(CH₂)ₚ-COOR₂ pour obtenir le diacide/ester de formule HOOC-(CH₂)ₚ-CH=CH-(CH₂)ₙ-COOR₂ puis l'ammoniation du diacides/diesters gras obtenu pour obtenir un dinitrile gras de formule NC-(CH₂)ₚ-CH=CH-(CH₂)ₙ-CN puis dans un deuxième stade on transforme ce dinitrile en un mélange de deux nitrile-acides de formules HOOC-(CH₂)ₚ-CN et HOOC-(CH₂)ₙ-CN par coupure oxydante et, enfin, dans un troisième stade on réduit par hydrogénation la fonction nitrile en fonction amine pour obtenir un mélange de composés de formules HOOC-(CH₂)ₚ-CH₂NH₂ et HOOC-(CH₂)ₙ-CH₂NH₂.

Dans une sixième variante de réalisation du procédé, lors du premier stade on réalise tout d'abord l'oxydation par fermentation de l'acide/ester gras de formule R₁-CH=CH-(CH₂)ₚ-COOR₂ pour obtenir le diacide/ester de formule HOOC-(CH₂)ₚ-CH=CH-(CH₂)ₙ-COOR₂, puis l'ammoniation du diacides/diesters gras obtenu pour obtenir un dinitrile gras de formule NC-(CH₂)ₚ-CH=CH-(CH₂)ₙ-CN puis dans un deuxième stade on transforme ce dinitrile en un mélange de deux nitrile-acide/esters de formules R₃OOC-[CH=CH]-(CH₂)ₚ-CN et R₃OOC-[CH=CH]-(CH₂)ₙ-CN par métathèse croisée avec l'acrylate d'alkyle CH₂=CH-COOR₃ et enfin dans un troisième stade on réduit simultanément par hydrogénation la double liaison et la fonction nitrile en amine pour obtenir un mélange de composés de formules R₃OOC-(CH₂)ₚ₊₂-CH₂NH₂ et R₃OOC-(CH₂)ₙ₊₂-CH₂NH₂.

Les conditions opératoires des différentes réactions mises en jeu sont connues et décrites dans l'état de l'art.

Le schéma réactionnel de la synthèse des nitriles à partir des acides peut se résumer de la façon suivante.

R-COOH + NH₃ → [R-COO⁻NH₄⁺] → [R-CONH₂] + H₂O → RCN + H₂O

Le procédé peut être conduit en batch en phase liquide ou gazeuse ou en continu en phase gazeuse. La réaction est conduite à température élevée > 250 °C et présence d'un catalyseur qui est généralement un oxyde métallique et le plus fréquemment l'oxyde de zinc. L'élimination en continu de l'eau formée en entraînant de surcroît l'ammoniac qui n'a pas réagi permet un achèvement rapide de la réaction.

Lorsque le procédé met en oeuvre une étape d'oxydation par fermentation, on utilise un micro-organisme, tel qu'une bactérie, un champignon ou une levure permettant l'oxydation de l'acide ou ester gras de la charge. On utilisera de préférence des micro-organismes contenant des enzymes de type *Oxygenase* susceptibles d'oxyder la charge en formant une fonction trivalente de type acide -COOH ou ester -COOR. Cette fermentation pourra par exemple être réalisée en présence d'une souche de *Candida tropicalis* contenant les enzymes Cytochrome P450 Monooxygenase telles que celles décrites dans la publication de W. H. Eschenfeldt et al « Transformation of fatty Acids Catalyzed by Cytochrome P450 Monooxygenase Enzymes of Candida tropicalis » paru dans Applied and Environmental Microbiology, Oct. 2003 p. 5992-5999 et les brevets FR 2,445,374 US4,474,882, US 3,823,070, US 3,912,586, US 6,660,505, US 6,569,670 et 5,254,466.

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004 (Plenum Publishing).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que Rhénium, Molybdène et Tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Dans le procédé de l'invention, tout catalyseur de métathèse actif et sélectif pourra être utilisé. On utilisera cependant de préférence des catalyseurs à base de ruthénium.

La réaction de métathèse croisée avec le composé de type acrylate est réalisée dans des conditions parfaitement connues. La température de réaction est comprise entre 20 et 100 °C à une pression proche de la pression atmosphérique (1 à 10 bars) en présence d'un catalyseur à base de ruthénium par exemple. Le temps de réaction est choisi en fonction des réactifs mis en oeuvre et pour atteindre au plus près l'équilibre de la réaction

Les catalyseurs au ruthénium sont choisis de préférence parmi les catalyseurs chargés ou non-chargés de formule générale :

(X1)a (X2)bRu(carbène C) (L1)c(L2)d

dans laquelle :
- a, b, c, d sont des nombres entiers avec a et b égaux à 0, 1 ou 2 ; c et d égaux à 0, 1, 2, 3 ou 4 ;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénates, les amidures, le tosylate, l'hexafluorophosphate, le tétrafluoroborate, le bis-triflylamidure, le tétraphénylborate et dérivés. X1 ou X2 peuvent être liés à Y1 ou Y2 ou au (carbène C) de façon à former un ligand bidenté (ou chélate) sur le ruthénium ; et
- L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique L1 ou L2 peuvent être liés au "carbène C " de façon à former un ligand bidenté ou chélate,

Le "carbène C" pourra être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHR ou allénylidènes Ru=C=C=CR1R2 ou indénylidènes.

Un groupe fonctionnel permettant d'améliorer la rétention du complexe du ruthénium dans le liquide ionique peut être gréffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

La réaction de coupure oxydante de la double liaison, qui conduit à la formation de la fonction acide sur les deux carbones de la double liaison, est également en elle-même connue.

Elle peut être réalisée au moyen d'un oxydant fort tel que KMnO₄ sous forme concentrée et à la chaleur ainsi que cela est décrit dans « Organic Chemistry » de L.G. Wade Jr. 5th Edition Chapter 8 Reactions of Alkenes.

La coupure oxydante peut être effectuée avec de l'eau oxygénée comme décrit dans le brevet GB 743491. L'article de F. Drawert et al. dans Chem. Mikrobiol. Technol. Lebensm. 1, 158-159 (1972) décrit une voie alternative par irradiation de l'huile de tournesol. D'autre part l'article de G.S. Zhang et al. dans Chinese Chemical Letters, Vol 5, N°2, pp105-108 de 1994, indique qu'il est possible d'effectuer la coupure oxydante à partir du diol correspondant à l'acide oléique (voir Entrée 29 du tableau). Cette coupure oxydante est effectuée en utilisant le Chlorochromate d'ammonium comme oxydant. Le diol quant à lui, est obtenu par époxydation de l'acide oléique suivie d'une hydrolyse du pont époxy.

Elle peut être réalisée par d'autres oxydants tels que l'eau oxygénée et plus particulièrement l'ozone.

De nombreux travaux ont été conduits sur l'utilisation de l'ozone comme agent oxydant. Il est d'ailleurs mentionné dans l'ouvrage Angew. Chem. cité ci-dessus que la coupure oxydante de l'acide oléique en acides pélargonique et azélaique est la plus importante application industrielle de l'ozonolyse

Le brevet USP 2,813,113 notamment décrit un procédé d'ozonolyse oxydante d'un acide gras tel que l'acide oléique qui consiste dans une première étape à traiter l'acide par de l'oxygène associé à de l'ozone pour former des ozonides puis dans une deuxième étape à oxyder ces derniers par l'oxygène.

Dans ce type de réaction on n'utilise pas de composés bloquant le processus l'oxydation au stade des cétones ou aldéhydes dans ce qui est appelé l'ozonolyse réductrice qui a fait plus récemment l'objet de travaux importants.

L'étape de synthèse des ω-aminoacides(esters) gras à partir des nitrile-acides gras consiste en une hydrogénation classique. Les catalyseurs sont nombreux mais préférentiellement on utilise les Nickel et Cobalt de Raney. Afin de favoriser la formation de l'amine primaire, on opère avec une pression partielle d'ammoniac. Enfin, la réduction de la fonction nitrile en amine primaire est bien connue de l'homme de l'art.

Dans le procédé de l'invention, on peut traiter l'acide gras soit sous sa forme acide soit sous sa forme ester. Le passage d'une forme à l'autre, par méthanolyse, estérification ou hydrolyse, parfaitement banal ne constitue pas une transformation chimique au sens du procédé.

Tous les mécanismes détaillés ci-dessous illustrent, pour faciliter l'exposé, la synthèse des acides. Cependant, la métathèse est aussi efficace avec un ester et même plus efficace, le milieu étant généralement plus anhydre. De la même manière, les schémas illustrent des réactions avec l'isomère cis des acides (ou esters) ; les mécanismes sont aussi bien applicables aux isomères trans.

Le mécanisme réactionnel de cette réaction est illustrée par le schéma 1 ci-dessous

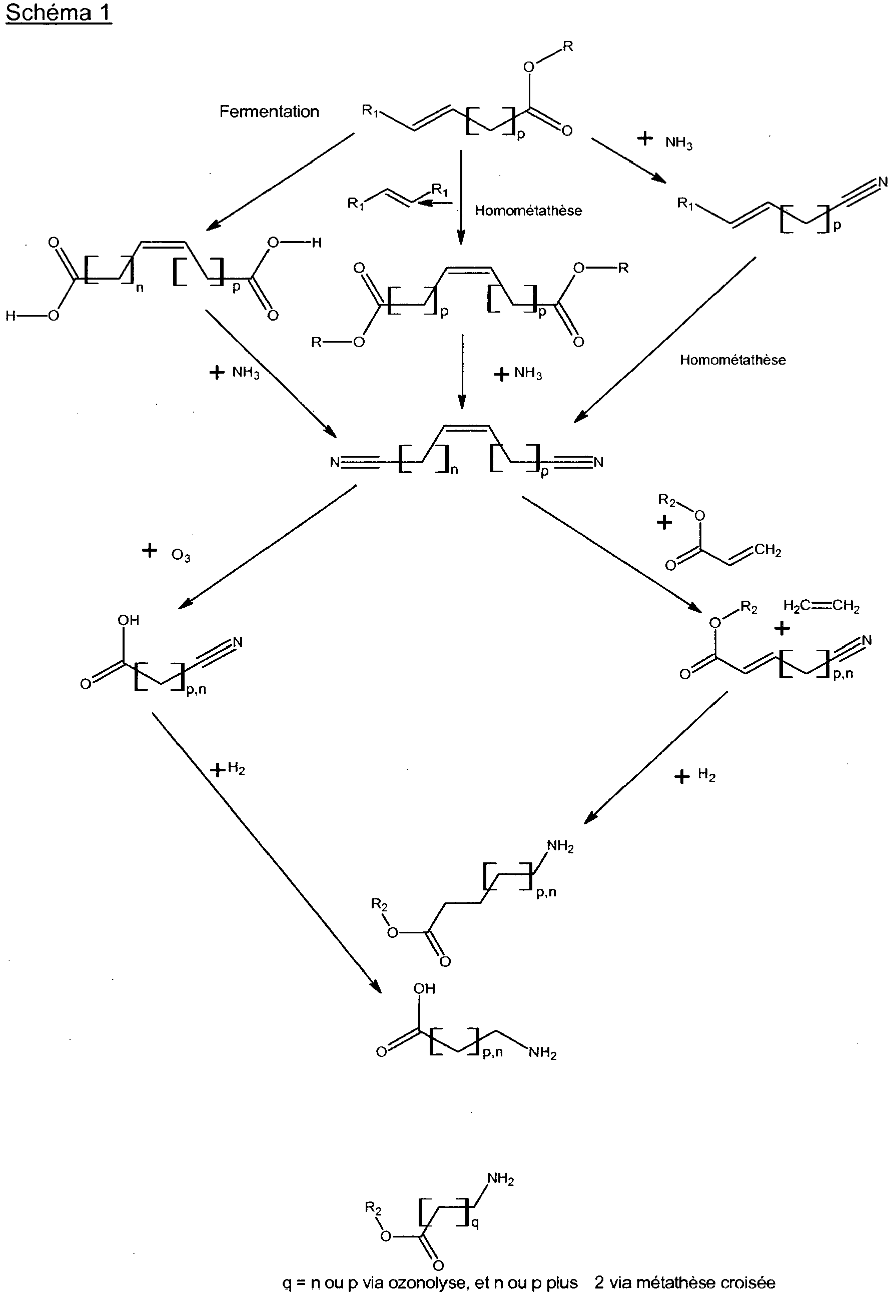

En mettant en oeuvre le procédé de l'invention, il sera possible de synthétiser toute la gamme des ω-aminoacides de l'amino-4-tétraoïque à l'amino-17-heptadécanoïque. L'acide amino-4-tétraoique peut être obtenu à partir des acides obtusilique, lindérique, et tsuzuique.

L'acide amino-5-pentanoïque peut être obtenu à partir des acides lauroléique, myristoléique, cis-5-eicosénoïque et physitérique.

L'acide amino-6-hexanoïque peut être obtenu à partir des acides obtusilique, lindérique, tsuzuique et pétrosélénique.

L'acide amino-7-heptanoïque peut être obtenu à partir des acides lauroléique, palmitoléique, myristoléique, physitérique, cis-5-eicosénoïque et vaccénique.

L'acide amino-8-octanoïque peut être obtenu à partir des acides obtusilique, lindérique et pétrosélénique.

L'acide amino-9-nonanoïque peut être obtenu à partir des acides caproléique, lauroléique, myristoléique, physitérique, palmitoléique, oléique, élaidique vaccénique, gadoléique, ricinoléique et érucique.

L'acide amino-10-décanoïque peut être obtenu à partir des acides lindérique et tsuzuique.

L'acide amino-11-undécanoïque peut être obtenu à partir des acides caproléique, myristoléique, physitérique, palmitoléique, oléique, élaidique, vaccénique, ricinoléique, lesquérolique, gadoléique et érucique.

L'acide amino-12-dodécanoïque peut être obtenu à partir des acides tsuzuique et pétrosélénique.

L'acide amino-13-tridécanoïque peut être obtenu à partir des acides vaccénique, gadoléique, lesquérolique et érucique.

L'acide amino-14-tétradécanoïque peut être obtenu à partir de l'acide pétrosélénique. L'acide amino-15-pentadécanoïque peut être obtenu à partir des acides érucique et , cis-5-eicosènoïque.

L'acide amino-17-heptadécanoïque peut être obtenu à partir de l'acide cis-5-eicosènoïque.

L'invention est illustrée par les exemples suivants.

### Exemple 1

Cet exemple illustre la première étape par fermentation de l'acide oléique produisant un diacide Dans cet exemple on utilisera une levure contenant au moins une enzyme *Oxygénase.* La levure sera cultivée à pH=7, dans un milieu d'eau désionisée contenant du sorbitol, des oligo-éléments, de l'urée et de l'acide oléique. Le mélange sera ensuite stérilisé à 120 °C pendant 15 minutes. Une souche de levure sera ensuite inoculée au milieu de culture. La culture sera maintenue à 30 °C. Une solution de soude sera ajoutée en continue pour maintenir le milieu à un pH de 7.0 à 7.5. Après 48 heures de culture, le diacide insaturé sera récupéré par extraction au diéthyléther. Après élimination du solvant par évaporation, on récupèrera des cristaux qui après recristallisation auront un point de fusion de 69 °C, c'est à dire équivalent à celui décrit pour le diacide 9-octadécènedioique.

### Exemple 2

Cet exemple illustre la première étape réalisée par homométathèse de l'acide oléique en diacide symétrique de formule HOOC-(CH2)7-CH=CH-(CH2)7-COOH l'acide 9-octadécénedioïque.

Pour cette étape, on utilise un catalyseurs de métathèse obtenu auprès de Sigma Aldrich, référence catalogue 569747 et répondant à la formule suivante benzylidene[1,3-bis(2,4,6-trimethylphenyl)-2-imidazolidinylidene] dichloro(tricyclohexyphosphine)ruthenium. Ce catalyseur est connu sous la dénomination de Grubbs seconde génération et Hoveyda-Grubbs seconde génération. Dans l'expérimentation on utilise 2,5 g d'ester d'acide gras de l'acide oléique (oléate de méthyle). Le tétradécane est utilisé comme étalon interne. Le mélange réactionnel est agité à 50 °C et dégazé par de l'argon. Le catalyseur (1% molaire) est ajouté à la solution, sans ajout de solvant. Les échantillons de produits de la réaction sont analysés par chromatographie. On obtient au bout d'une demi-heure de réaction une conversion de 98 % molaire avec un rendement de 100% d'homométathèse.

### Exemple 3

Cet exemple illustre l'étape d'ammoniation transformant le diacide insaturé issu de l'exemple 1 ou 2 en dinitrile insaturé.

La réaction d'ammoniation de l'acide 9-octadécénedioique pour former le dinitrile insaturé de formule NC-(CH₂)₇-CH=CH-(CH₂)₇-CN est conduite en batch avec introduction d'ammoniac en large excès molaire par rapport à l'acide et à une température de 300°C à la pression atmosphérique (en phase gazeuse), en présence d'un catalyseur d'oxyde de zinc. Le réacteur est équipé d'un condenseur à 100 °C. L'ammoniac est ainsi injecté en continu pendant 6 heures. L'élimination en continu de l'eau formée entraîne l'ammoniac en excès et permet un achèvement rapide de la réaction. Le rendement de la réaction est mesuré par chromatographie et est de 86 % par rapport à l'acide.

### Exemple 4

Cet exemple illustre la série de réactions ammoniation de l'acide oléique suivie d'une d'homométathèse du nitrile insaturé, ainsi obtenu, en dinitrile insaturé.

La réaction d'ammoniation de l'acide oléique est conduite dans des conditions analogues à celles de l'exemple 3 avec l'acide 9-octadécénedioique.

La réaction de métathèse est conduite à la pression atmosphérique à 80 °C en présence d'un catalyseur à base de Ruthénium [RuCl₂(=CHPh)(IMesH₂)(PCy₃)] en utilisant le toluène comme solvant. Les rendements sont déterminés par analyse chromatographique. Au terme de la réaction, 6 heures, on sépare l'oléfine en C18 du dinitrile par distillation sous vide.

### Exemple 5

Cet exemple illustre la coupure oxydante d'un dinitrile insaturé (symétrique ou non) de formule NC-(CH₂)₇-CH=CH -(CH₂)₇-CN par ozonolyse oxydante pour former le nitrile acide de formule CN-(CH₂)₇-COOH.

De l'ozone obtenue par un générateur d'ozone Welsbach T-408, est bullée dans 25 ml de pentane jusqu'à ce qu'une couleur bleue soit observée. La solution de pentane est maintenue à -70°C avec un bain d'acétone-carboglace. 20 mg de dinitrile, dissout dans 5 ml de pentane refroidis à 0 °C sont ajoutés à la solution d'ozone. L'excès d'ozone est ensuite éliminé et la couleur bleue disparaît. Après 5 minutes, le pentane est évaporé par un flux d'azote sec. Pendant cette étape la température de la solution est maintenue inférieure à 0°C. Après évaporation du pentane, 3 ml de méthanol refroidi à -70 °C sont ajoutés au réacteur, tout en le réchauffant pour permettre la dissolution de l'ozonide.

### Exemple 6

Cet exemple illustre la réaction de métathèse croisée entre le dinitrile de formule issu de l'étape des exemples 2 et 4 avec l'acrylate de méthyle pour former le nitrile acide de formule NC-(CH₂)₇-CH=CH-COOH.

Pour cette étape on utilise un catalyseur obtenu auprès de Sigma Aldrich, sous la référence catalogue 569755 connu comme un catalyseur Grubbs seconde génération et Hoveyda-Grubbs seconde génération. Sa formule est la suivante : (1,3-bis(2,4,6-trimethylphenyl)-2-imidazolidinylidene]dichloro(o-isopropoxy-phenylmethylene)ruthenium.

Dans l'expérimentation, 2,5 g de dinitrile oléique gras sont mélangés avec un excès d'acrylate de méthyle (rapport molaire 10/1). Le tétradécane est utilisé comme étalon interne. Le mélange réactionnel est agité à 50 °C et dégazé par de l'argon. Le catalyseur (0,1% molaire) est ajouté à la solution, sans ajout de solvant. Les échantillons de produits de la réaction sont analysés par chromatographie.. On obtient au bout d'une demi-heure de réaction une conversion de 99 % molaire avec un rendement de 99% de métathèse croisée.

### Exemple 7

Cet exemple illustre l'hydrogénation de la double liaison et de la fonction nitrile. Celle-ci est réalisée en présence d'un catalyseur constitué d'un nickel de Raney.

1 g de nitrile acide de formule NC-(CH₂)₇-COOH obtenu conformément à l'exemple 3 est estérifié avec le méthanol. On introduit dans un réacteur 1g de l'acide-nitrile, 1,2 g de méthanol, 1,2 g de benzène et quelques gouttes d'acide sulfurique concentré. L'azéotrope eau-alcool-benzène est éliminé en tête de colonne. De l'acide sulfurique est ajouté en continu pour maintenir l'avancement de la réaction. Ensuite le benzène et l'alcool sont flashés pour récupérer le nitrile ester : 1,02 g.

Le nitrile-ester synthétisé est placé dans un autoclave agité de 15 ml et on y ajoute 2,5 g d'éthanol à 96 %, 2,5 g d'ammoniac liquide et 0,125 g de catalyseur nickel de Raney contenant 3 % poids de cobalt. Le mélange est chauffé à 90 °C, sous 150 bars d'hydrogène (pression totale 210 bars) et pendant 4 heures. L'ester méthylique est distillé sous vide de 0,5 mm de mercure. Un distillat clair de 0,97 g est récupéré. Il contient 90 % d'amino ester.

### Exemple 8

Cet exemple illustre la métathèse croisée entre l'oléonitrile et l'acrylate de méthyle selon le shéma réactionnel suivant :

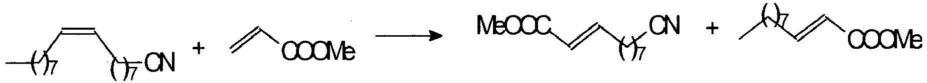

Dans un tube de Schlenk de 50 ml purgé à l'azote, on charge 132 mg de 9-octadécènenitrile (0,5 mmol), 172 mg d'acrylate de méthyle (2 mmol) et 10 ml de toluène distillé sur sodium benzophénone. On chauffe à 100°C, puis sous agitation magnétique, on ajoute avec une seringue et un pousse seringue 0,15 mg (2,5.10-4 mmol) de catalyseur de Hoveyda-Grubbs de seconde génération (Aldrich) dissout dans 2 ml de toluène sur une période de 4h. A la fin de l'ajout, on laisse réagir 2 heures à 100°C. Le mélange réactionnel est analysé par chromatographie en phase gaz :
- La conversion du 9-octadécènenitrile est de 93%.
- Le rendement en 10-cyano-2-décénoate de méthyle est de 80%.

Le rendement est exprimé en nombre de moles de nitrile-ester obtenu par rapport au nombre de moles de nitrile C11 engagé.

## Revendications

1. Procédé de synthèse d'un ω-aminoacide(ester) de formule ROOC-(CH₂)_{q}-CH₂NH₂ dans laquelle R est H ou un radical alkyle comportant de 1 à 4 atomes de carbone et q un indice entier égal soit à p ou à p+2, soit à n ou n+2, compris entre 2 et 15, à partir d'un acide(ester) gras mono-insaturé de formule R₁-CH=CH-(CH₂)ₚ-COOR₂ dans laquelle R₁ est H ou un radical alkyle comportant de 4 à 14 atomes de carbone et le cas échéant une fonction hydroxyle, R₂ est H ou un radical alkyle comprenant de 1 à 4 atomes de carbone et p un indice entier compris entre 2 et 11, comportant une étape réactionnelle d'ammoniation conduisant à la transformation de la fonction carbonyle en fonction nitrile **caractérisé en ce que** :
- dans un premier stade on transforme l'acide/ester gras insaturé en un dinitrile insaturé de formule NC-(CH₂)ₚ-CH=CH-(CH₂)ₙ-CN dans laquelle n est un entier compris entre 3 et 13 dépendant de la nature du radical R₁, en deux étapes successives, la première étape étant soit une homométathèse de l'acide gras conduisant au diacide insaturé symétrique de formule R₂OOC-(CH₂)ₚ-CH=CH-(CH₂)ₚ-soit une fermentation de cet acide/ester conduisant à un diacide insaturé de formule HOOC-(CH₂)ₚ-CH=CH-(CH₂)ₙ-COOH et la seconde une ammoniation des acides puis,
- dans un deuxième stade on transforme ce dinitrile insaturé en un nitrile-acide/ester de formule R₃OOC-[CH=CH]ₓ-(CH₂)_{p,n}-CN dans laquelle R₃ est H ou un radical alkyle comportant de 1 à 4 atomes de carbone, x est 0 ou 1, « p,n » signifiant que l'indice est soit p, soit n selon la voie choisie lors du premier stade, transformation réalisée, soit par coupure oxydante du dinitrile insaturé, soit par une réaction de métathèse croisée du dinitrile insaturé avec un acrylate de formule CH₂=CH-COOR₃ et,
- dans un troisième stade à hydrogéner le nitrile acide en ω-aminoacide(ester) de formule COOR-(CH₂)_{q}-CH₂NH₂.

2. Procédé selon la revendication 1 **caractérisé en ce que** lors du premier stade on réalise tout d'abord l'homométathèse de l'acide(ester) gras de formule R₁-CH=CH-(CH₂)ₚ-COOR₂, puis l'ammoniation du diacides/diesters gras obtenu pour obtenir un dinitrile gras, puis dans un deuxième stade on transforme ce dinitrile en nitrile-acide de formule HOOC-(CH₂)ₚ-CN par coupure oxydante et, enfin, dans un troisième stade on réduit par hydrogénation la fonction nitrile en fonction amine pour obtenir le composé de formule ROOC-(CH₂)ₚ-CH₂NH₂.

3. Procédé selon la revendication 1 **caractérisé en ce que** lors du premier stade on réalise tout d'abord l'homométathèse de l'acide(ester) gras de formule R₁-CH=CH-(CH₂)ₚ-COOR₂ pour obtenir le diacide/ester de formule R₂OOC-(CH₂)ₚ-CH=CH-(CH₂)ₚ-COOR₂ puis l'ammoniation du diacides/diesters gras obtenu pour obtenir un dinitrile gras, puis dans un deuxième stade on transforme ce dinitrile en nitrile-acide/ester de formule R₃OOC-[CH=CH]-(CH₂)ₚ-CN par métathèse croisée avec l'acrylate d'alkyle CH₂=CH-COO R₃ et enfin dans un troisième stade on réduit simultanément par hydrogénation la double liaison et la fonction nitrile en amine pour obtenir le composé de formule R₃OOC-(CH₂)ₚ₊₂-CH₂NH₂.

4. Procédé selon la revendication 1 **caractérisé en ce que** lors du premier stade on réalise tout d'abord l'ammoniation de l'acide(ester) gras de formule R₁-CH=CH-(CH₂)ₚ-COOR₂ conduisant au nitrile correspondant puis la transformation par homométathèse du nitrile en dintrile gras insaturé de formule NC-(CH₂)ₚ-CH=CH-(CH₂)ₚ-CN, puis on transforme ce dinitrile en nitrile-acide de formule HOOC-(CH₂)ₚ-CN par coupure oxydante et, enfin, dans un troisième stade on réduit par hydrogénation la fonction nitrile en fonction amine pour obtenir le composé de formule HOOC-(CH₂)ₚ-CH₂NH₂.

5. Procédé selon la revendication 1 **caractérisé en ce que** lors du premier stade on réalise tout d'abord l'ammoniation de l'acide(ester) gras de formule R₁-CH=CH-(CH₂)ₚ-COOR₂ conduisant au nitrile correspondant puis la transformation par homométathèse du nitrile en dintrile gras insaturé de formule NC-(CH₂)ₚ-CH=CH-(CH₂)ₚ-CN, puis dans un deuxième stade on transforme ce dinitrile en nitrile-acideester de formule R₃OOC-[CH=CH] -(CH₂)ₚ-CN par métathèse croisée avec l'acrylate d'alkyle CH₂=CH-COOR₃ et enfin dans un troisième stade on réduit simultanément par hydrogénation la double liaison et la fonction nitrile en amine pour obtenir le composé de formule R₃OOC-(CH₂)ₚ₊₂-CH₂NH₂

6. Procédé selon la revendication 1 **caractérisé en ce que** lors du premier stade on réalise tout d'abord l'oxydation par fermentation de l'acide/ester gras de formule R₁-CH=CH-(CH₂)ₚ-COOR₂ pour obtenir le diacide/ester de formule HOOC-(CH₂)ₚ-CH=CH-(CH₂)ₙ-COOR₂ puis l'ammoniation du diacides/diesters gras obtenu pour obtenir un dinitrile gras de formule NC-(CH₂)ₚ-CH=CH-(CH₂)ₙ-CN puis dans un deuxième stade on transforme ce dinitrile en un mélange de deux nitrile-acide/esters de formules HOOC-(CH₂)ₚ-CN et HOOC-(CH₂)ₙ-CN par coupure oxydante et, enfin, dans un troisième stade on réduit par hydrogénation la fonction nitrile en fonction amine pour obtenir un mélange de composés de formules HOOC-(CH₂)ₚ-CH₂NH₂ et HOOC-(CH₂)ₙ-CH₂NH₂.

7. Procédé selon la revendication 1 **caractérisé en ce que** lors du premier stade on réalise tout d'abord l'oxydation par fermentation de l'acide/ester gras de formule R₁-CH=CH-(CH₂)ₚ-COOR₂ pour obtenir le diacide/ester de formule HOOC-(CH₂)ₚ-CH=CH-(CH₂)ₙ-COOR₂, puis l'ammoniation du diacides/diesters gras obtenu pour obtenir un dinitrile gras de formule NC-(CH₂)ₚ-CH=CH-(CH₂)ₙ-CN puis dans un deuxième stade on transforme ce dinitrile en un mélange de deux nitrile-acide/esters de formules R₃OOC-[CH=CH]-(CH₂)ₚ-CN et R₃OOC-[CH=CH]-(CH₂)ₙ-CN par métathèse croisée avec l'acrylate d'alkyle CH₂=CH-COOR₃ et enfin dans un troisième stade on réduit simultanément par hydrogénation la double liaison et la fonction nitrile en amine pour obtenir un mélange de composés de formules R₃OOC-(CH₂)ₚ₊₂-CH₂NH₂ et R₃OOC-(CH₂)ₙ₊₂-CH₂NH₂.

8. Procédé selon l'une des revendications 1 à 7 **caractérisé en ce que** les métathèses sont réalisées en présence d'un catalyseur à base de Ruthénium.

9. Procédé selon l'une des revendications 1 à 7 **caractérisé en ce que** l'oxydation par fermentation est réalisée de préférence au moyen de micro-organismes contenant des enzymes de type *Oxygenase.*

10. Procédé selon l'une des revendications 1 à 7 **caractérisé en ce que** la coupure oxydante est réalisée par ozonolyse.

11. Procédé selon l'une des revendications 1 à 7 **caractérisé en ce que** la réaction de métathèse croisée est réalisée avec l'acrylate de méthyle.

## Patentansprüche

1. Verfahren zur Synthese einer ω-Aminosäure bzw. eines ω-Aminosäureesters der Formel ROOC-(CH₂)_{q}-CH₂NH₂, worin R für H oder einen Alkylrest mit 1 bis 4 Kohlenstoffatomen steht und q für einen ganzzahligen Index, der gleich p oder gleich p+2 oder gleich n oder n+2 ist, zwischen 2 und 15 steht, aus einer einfach ungesättigten Fettsäure bzw. einem einfach ungesättigten Fettsäureester der Formel R₁-CH=CH-(CH₂)ₚ-COOR₂, worin R₁ für H oder einen Alkylrest mit 4 bis 14 Kohlenstoffatomen und gegebenenfalls einer Hydroxylfunktion steht, R₂ für H oder einen Alkylrest mit 1 bis 4 Kohlenstoffatomen steht und p für einen ganzzahligen Index zwischen 2 und 11 steht, umfassend einen Ammonierungsreaktionsschritt, der zur Umwandlung der Carbonylfunktion in eine Nitrilfunktion führt, **dadurch gekennzeichnet, dass**:
- man in einer ersten Stufe die ungesättigte Fettsäure bzw. den ungesättigten Fettsäureester in zwei aufeinanderfolgenden Schritten in ein ungesättigtes Dinitril der Formel NC-(CH₂)ₚ-CH=CH-(CH₂)ₙ-CN, worin n je nach der Beschaffenheit des Rests R₁ für eine ganze Zahl zwischen 3 und 13 steht, umwandelt, wobei es sich bei dem ersten Schritt entweder um eine Homometathese der Fettsäure, die zu der symmetrischen ungesättigten Disäure der Formel R₂OOC-(CH₂)ₚ-CH=CH-(CH₂)ₚ-COOR₂ führt, oder eine Fermentation dieser Säure bzw. dieses Esters, die zu einer ungesättigten Disäure der Formel HOOC-(CH₂)ₚ-CH=CH-(CH₂)ₙ-COOH führt, handelt und es sich bei dem zweiten Schritt um eine Ammonierung der Säuren handelt, dann
- man in einer zweiten Stufe dieses ungesättigte Dinitril in eine Nitrilsäure bzw. einen Nitrilester der Formel R₃OOC-[CH=CH]ₓ-(CH₂)_{p,n}-CN, worin R₃ für H oder einen Alkylrest mit 1 bis 4 Kohlenstoffatomen steht, x für 0 oder 1 steht und "p,n" bedeutet, dass der Index je nach dem in der ersten Stufe gewählten Weg entweder gleich p oder gleich n ist, umwandelt, wobei diese Umwandlung entweder durch oxidative Spaltung des ungesättigten Dinitrils oder durch eine Kreuzmetathesereaktion des ungesättigten Dinitrils mit einem Acrylat der Formel CH₂=CH-COOR₃ durchgeführt wird, und
- man in einer dritten Stufe die Nitrilsäure zu einer ω-Aminosäure bzw. zu einem ω-Aminosäureester der Formel COOR-(CH₂)_{q}-CH₂NH₂ hydriert.

2. Verfahren nach Anspruch 1, **dadurch gekennzeichnet, dass** man in der ersten Stufe zunächst die Homometathese der Fettsäure bzw. des Fettsäureesters der Formel R₁-CH=CH-(CH₂)ₚ-COOR₂ und dann die Ammonierung der erhaltenen Fettdisäuren bzw. Fettdiester zu einem Fettdinitril durchführt, dann in einer zweiten Stufe dieses Dinitril durch oxidative Spaltung in die Nitrilsäure der Formel HOOC-(CH₂)ₚ-CN umwandelt und schließlich in einer dritten Stufe durch Hydrierung die Nitrilfunktion zu einer Aminfunktion reduziert, was die Verbindung der Formel ROOC-(CH₂)ₚ-CH₂NH₂ ergibt.

3. Verfahren nach Anspruch 1, **dadurch gekennzeichnet, dass** man in der ersten Stufe zunächst die Homometathese der Fettsäure bzw. des Fettsäureesters der Formel R₁-CH=CH-(CH₂)ₚ-COOR₂ zu der Disäure bzw. dem Ester der Formel R₂OOC- (CH₂)ₚ-CH=CH- (CH₂) ₚ-COOR₂ und dann die Ammonierung der erhaltenen Fettdisäuren bzw. Fettdiester zu einem Fettdinitril durchführt, dann in einer zweiten Stufe dieses Dinitril durch Kreuzmetathese mit dem Alkylacrylat der Formel CH₂=CH-COOR₃ in die Nitrilsäure bzw. den Ester der Formel R₃OOC-[CH=CH]-(CH₂)ₚ-CN umwandelt und schließlich in einer dritten Stufe gleichzeitig durch Hydrierung die Doppelbindung und die Nitrilfunktion zum Amin reduziert, was die Verbindung der Formel R₃OOC-(CH₂)ₚ₊₂-CH₂NH₂ ergibt.

4. Verfahren nach Anspruch 1, **dadurch gekennzeichnet, dass** man in der ersten Stufe zunächst die Ammonierung der Fettsäure bzw. des Fettsäureesters der Formel R₁-CH=CH-(CH₂)ₚ-COOR₂ zu dem entsprechenden Nitril und dann die Umwandlung des Nitrils durch Homometathese zu dem ungesättigten Fettdinitril der Formel NC- (CH₂) ₚ-CH=CH- (CH₂) ₚ-CN durchführt, dann in einer zweiten Stufe dieses Dinitril durch oxidative Spaltung in die Nitrilsäure der Formel HOOC-(CH₂)ₚ-CN umwandelt und schließlich in einer dritten Stufe durch Hydrierung die Nitrilfunktion zu einer Aminfunktion reduziert, was die Verbindung der Formel HOOC-(CH₂)ₚ-CH₂NH₂ ergibt.

5. Verfahren nach Anspruch 1, **dadurch gekennzeichnet, dass** man in der ersten Stufe zunächst die Ammonierung der Fettsäure bzw. des Fettsäureesters der Formel R₁-CH=CH-(CH₂)ₚ-COOR₂ zu dem entsprechenden Nitril und dann die Umwandlung des Nitrils durch Homometathese zu dem ungesättigten Fettdinitril der Formel NC-(CH₂)ₚ-CH=CH-(CH₂)ₚ-CN durchführt, dann in einer zweiten Stufe dieses Dinitril durch Kreuzmetathese mit dem Alkylacrylat der Formel CH₂=CH-COOR₃ in die Nitrilsäure bzw. den Nitrilester der Formel R₃OOC-[CH=CH]-(CH₂)ₚ-CN umwandelt und schließlich in einer dritten Stufe gleichzeitig durch Hydrierung die Doppelbindung und die Nitrilfunktion zum Amin reduziert, was die Verbindung der Formel R₃OOC-(CH₂)ₚ+2-CH₂NH₂ ergibt.

6. Verfahren nach Anspruch 1, **dadurch gekennzeichnet, dass** man in der ersten Stufe zunächst die Oxidation der Fettsäure bzw. des Fettsäureesters der Formel R₁-CH=CH-(CH₂)ₚ-COOR₂ durch Fermentation zu der Disäure bzw. dem Ester der Formel HOOC-(CH₂)ₚ-CH=CH-(CH₂)ₙ-COOR₂ und dann die Ammonierung der erhaltenen Fettdisäuren bzw. Fettdiester zu einem Fettdinitril der Formel NC-(CH₂)ₚ-CH=CH-(CH₂)ₙ-CN durchführt, dann in einer zweiten Stufe dieses Dinitril durch oxidative Spaltung in ein Gemisch von zwei Nitrilsäuren bzw. Nitrilestern der Formeln HOOC-(CH₂)ₚ-CN und HOOC-(CH₂)ₙ-CN umwandelt und schließlich in einer dritten Stufe durch Hydrierung die Nitrilfunktion zu einer Aminfunktion reduziert, was ein Gemisch der Verbindungen der Formeln HOOC-(CH₂)ₚ-CH₂NH₂ und HOOC-(CH₂)ₙ-CH₂NH₂ ergibt.

7. Verfahren nach Anspruch 1, **dadurch gekennzeichnet, dass** man in der ersten Stufe zunächst die Oxidation der Fettsäure bzw. des Fettsäureesters der Formel R₁-CH=CH-(CH₂)ₚ-COOR₂ durch Fermentation zu der Disäure bzw. dem Ester der Formel HOOC-(CH₂)ₚ-CH=CH-(CH₂)ₙ-COOR₂ und dann die Ammonierung der erhaltenen Fettdisäuren bzw. Fettdiester zu einem Fettdinitril der Formel NC-(CH₂)ₚ-CH=CH-(CH₂)ₙ-CN durchführt, dann in einer zweiten Stufe dieses Dinitril durch Kreuzmetathese mit dem Alkylacrylat der Formel CH₂=CH-COOR₃ in ein Gemisch von zwei Nitrilsäuren bzw. Nitrilestern der Formeln R₃OOC-[CH=CH]-(CH₂)ₚ-CN und R₃OOC-[CH=CH]-(CH₂)ₙ-CN umwandelt und schließlich in einer dritten Stufe gleichzeitig durch Hydrierung die Doppelbindung und die Nitrilfunktion zum Amin reduziert, was ein Gemisch der Verbindungen der Formeln R₃OOC-(CH₂)ₚ₊₂CH₂NH₂ und R₃OOC-(CH₂)ₙ₊₂-CH₂NH₂ ergibt.

8. Verfahren nach einem der Ansprüche 1 bis 7, **dadurch gekennzeichnet, dass** man die Metathesen in Gegenwart eines Katalysators auf Basis von Ruthenium durchführt.

9. Verfahren nach einem der Ansprüche 1 bis 7, **dadurch gekennzeichnet, dass** man die Oxidation durch Fermentation vorzugsweise mit Hilfe von Mikroorganismen, die Enzyme von *Oxygenase*-Typ enthalten, durchführt.

10. Verfahren nach einem der Ansprüche 1 bis 7, **dadurch gekennzeichnet, dass** man die oxidative Spaltung durch Ozonolyse durchführt.

11. Verfahren nach einem der Ansprüche 1 bis 7, **dadurch gekennzeichnet, dass** man die Kreuzmetathesereaktion mit Ethylacrylat durchführt.

## Claims

1. Process for the synthesis of an ω-amino acid (ester) of formula ROOC- (CH₂) _{q}-CH₂-NH₂, in which R is H or an alkyl radical comprising from 1 to 4 carbon atoms and q is an integral index equal either to p or to p+2 or to n or n+2, of between 2 and 15, starting from a monounsaturated fatty acid (ester) of formula R₁-CH-CH-(CH₂)ₚ-COOR₂, in which R₁ is H or an alkyl radical comprising from 4 to 14 carbon atoms and, if appropriate, a hydroxyl functional group, R₂ is H or an alkyl radical comprising from 1 to 4 carbon atoms and p is an integral index of between 2 and 11, comprising an ammoniation reaction stage resulting in the conversion of the carbonyl functional group to a nitrile functional group, **characterized in that**:
- in a first stage, the unsaturated fatty acid/ester is converted to an unsaturated dinitrile of formula NC-(CH₂)ₚ-CH=CH-(CH₂)ₙ-CN, in which n is an integer of between 3 and 13, depending on the nature of the R₁ radical, in two successive stages, the first stage being either a homometathesis of the fatty acid, resulting in the symmetrical unsaturated diacid of formula R₂OOC-(CH₂)ₚ-CH-CH-(CH₂)ₚ-COOR₂, or a fermentation of this acid/ester, resulting in an unsaturated diacid of formula HOOC-(CH₂)ₚ-CH=CH-(CH₂)ₙ-COOH, and the second stage being an ammoniation of the acids, then,
- in a second stage, this unsaturated dinitrile is converted to an acid/ester nitrile of formula R₃OOC-[CH=CH]ₓ-(CH₂)_{p,n}-CN, in which R₃ is H or an alkyl radical comprising from 1 to 4 carbon atoms, x is 0 or 1 and "p,n" means that the index is either p or n, according to the route chosen during the first stage, which conversion is carried out either by oxidative cleavage of unsaturated dinitrile or by a cross metathesis reaction of the unsaturated dinitrile with an acrylate of formula CH₂=CH-COOR₃, and,
- in a third stage, the acid/ester nitrile is hydrogenated to give an ω-amino acid (ester) of formula COOR- (CH₂)_{q}-CH₂NH₂.

2. Process according to Claim 1, **characterized in that**, during the first stage, the homometathesis of the fatty acid (ester) of formula R₁-CH=CH-(CH₂)ₚ-COOR₂ is first of all carried out and then the ammoniation of the fatty diacids/diesters obtained is carried out in order to obtain a fatty dinitrile, then, in a second stage, this dinitrile is converted to an acid nitrile of formula HOOC-(CH₂)ₚ-CN by oxidative cleavage and, finally, in a third stage, the nitrile functional group is reduced by hydrogenation to give an amine functional group, in order to obtain the compound of formula ROOC-(CH₂)ₚ-CH₂NH₂.

3. Process according to Claim 1, **characterized in that**, during the first stage, the homometathesis of the fatty acid (ester) of formula R₁-CH-CH-(CH₂)ₚ-COOR₂ is first of all carried out, in order to obtain the diacid/ester of formula R₂OOC-(CH₂)ₚ-CH=CH-(CH₂)ₚ-COOR₂, and then the ammoniation of the fatty diacids/diesters obtained is carried out, in order to obtain a fatty dinitrile, then, in a second stage, this dinitrile is converted to an acid/ester nitrile of formula R₃OOC-[CH=CH]-(CH₂)ₚ-CN by cross metathesis with the alkyl acrylate CH₂=CH-COOR₃ and, finally, in a third stage, the double bond and the nitrile functional group are simultaneously reduced by hydrogenation to give an amine, in order to obtain the compound of formula R₃OOC- (CH₂)ₚ₊₂-CH₂NH₂.

4. Process according to Claim 1, **characterized in that**, during the first stage, the ammoniation of the fatty acid (ester) of formula R₁-CH=CH-(CH₂)ₚ-COOR₂ is first of all carried out, resulting in the corresponding nitrile, and then the conversion by homometathesis of the nitrile to give an unsaturated fatty dinitrile of formula NC-(CH₂)ₚ-CH=CH-(CH₂)ₚ-CN is carried out, then, in a second stage, this dinitrile is converted to an acid nitrile of formula HOOC-(CH₂)ₚ-CN by oxidative cleavage and, finally, in a third stage, the nitrile functional group is reduced to an amine functional group by hydrogenation, in order to obtain the compound of formula HOOC-(CH₂)ₚ-CH₂NH₂.

5. Process according to Claim 1, **characterized in that**, during the first stage, the ammoniation of the fatty acid (ester) of formula R₁-CH=CH-(CH₂)ₚ-COOR₂ is first of all carried out, resulting in the corresponding nitrile, and then the conversion by homometathesis of the nitrile to give an unsaturated fatty dinitrile of formula NC-(CH₂)ₚ-CH-CH-(CH₂)ₚ-CN is carried out, then, in a second stage, this dinitrile is converted to an acid/ester nitrile of formula R₃OOC-[CH=CH]-(CH₂)ₚ-CN by cross metathesis with the alkyl acrylate CH₂=CH-COOR₃ and, finally, in a third stage, the double bond and the nitrile functional group are simultaneously reduced by hydrogenation to give an amine, in order to obtain the compound of formula R₃OOC- (CH₂)ₚ₊₂-CH₂NH₂.

6. Process according to Claim 1, **characterized in that**, during the first stage, the oxidation by fermentation of the fatty acid/ester of formula R₁-CH=CH-(CH₂)ₚ-COOR₂ is first of all carried out, in order to obtain the diacid/ester of formula HOCC-(CH₂)ₚ-CH-CH-(CH₂)-COOR₂, and then the ammoniation of the fatty diacids/diesters obtained is carried out, in order to obtain a fatty dinitrile of formula NC-(CH₂)ₚ-CH=CH-(CH₂)ₙ-CN, then, in a second stage, this dinitrile is converted to a mixture of two acid/ester nitriles of formulae HOOC-(CH₂)ₚ-CN and HOOC-(CH₂)ₙ-CN by oxidative cleavage and, finally, in a third stage, the nitrile functional group is reduced by hydrogenation to an amine functional group, in order to obtain a mixture of compounds of formulae HOOC-(CH₂)ₚ-CH₂NH₂ and HOOC-(CH₂)ₙ-CH₂NH₂.

7. Process according to Claim 1, **characterized in that**, during the first stage, the oxidation by fermentation of the fatty acid/ester of formula R₁-CH=CH-(CH₂)ₚ-COOR₂ is first of all carried out, in order to obtain the diacid/ester of formula HOOC-(CH₂)ₚ-CH=CH-(CH₂)ₙ-COOR₂, and then the ammoniation of the fatty diacids/diesters obtained is carried out, in order to obtain a fatty dinitrile of formula NC-(CH₂)ₚ-CH-CH-(CH₂)ₙ-CN, then, in a second stage, this dinitrile is converted to a mixture of two acid/ester nitriles of formulae R₃OOC-[CH=CH]-(CH₂)ₚ-CN and R₃OOC-[CH=CH]-(CH₂)ₙ-CN by cross metathesis with the alkyl acrylate CH₂=CH-COOR₃ and, finally, in a third stage, the double bond and the nitrile functional group are simultaneously reduced by hydrogenation to give an amine, in order to obtain a mixture of compounds of formulae R₃OOC-(CH₂) ₚ₊₂-CH₂NH₂ and R₃OOC-(CH₂)ₙ₊₂-CH₂NH₂.

8. Process according to one of Claims 1 to 7, **characterized in that** the metatheses are carried out in the presence of a ruthenium-based catalyst.

9. Process according to one of Claims 1 to 7, **characterized in that** oxidation by fermentation is preferably carried out by means of microorganisms comprising enzymes of *Oxygenase* type.

10. Process according to one of Claims 1 to 7, **characterized in that** the oxidative cleavage is carried out by ozonolysis.

11. Process according to one of Claims 1 to 7, **characterized in that** the cross metathesis reaction is carried out with methyl acrylate.
